# EUROPEAN PATENT SPECIFICATION

(11) **EP 1 841 863 B1**
(45) Date of publication and mention of the grant of the patent: **04.08.2010**
(21) Application number: 06700982.9
(22) Date of filing: 13.01.2006
(51) Int. Cl.: C12N 9/64

(54) **METHOD FOR PURIFICATION OF FACTOR VII**
VERFAHREN ZUR REINIGUNG VON FACTOR VII
METHODE DE PURIFICATION DE FACTEUR VII

(30) Priority: 14.01.2005 US 644126 P
(43) Date of publication of application: 10.10.2007
(73) Proprietor: Bayer HealthCare LLC, Berkeley CA 94710 (US)
(72) Inventor: JENSEN, Rikkle Bolding, DK-4050 Skibby (DK); NYGAARD, Frank Bech, DK-3050 Humblebæk (DK)
(74) Representative: Burkert, Frank
(86) International application number: PCT/DK2006/000024
(87) International publication number: WO 2006/074664

(56) References cited:
- US-A- 4 470 969
- US-A- 4 473 553
- US-A- 4 637 932
- US-A- 5 700 914

## Description

### FIELD OF THE INVENTION

The present invention is directed to a method for purification of Factor VII protein using hydroxyapatite.

### BACKGROUND OF THE INVENTION

Factor VII (FVII), an important protein in the blood coagulation cascade, is a vitamin K-dependent plasma protein synthesized in the liver and secreted into the blood as a single-chain glycoprotein with a molecular weight of 53 kDa. The FVII zymogen is converted into an activated form (FVIIa) by proteolytic cleavage at a single site, R152-I153, resulting in two chains linked by a single disulfide bridge. Recombinant human FVIIa is commercially available from Novo Nordisk under the name NovoSeven® and is used for the treatment of bleeding episodes, e.g. in hemophilia or trauma. Recombinantly produced variants of human FVII have also been reported.

Purification of recombinant Factor VII (rFVII) or recombinant activated Factor VII (rFVIIa) is generally carried out using a combination of ion exchange and immuno-affinty chromatography based on a Ca²⁺-dependent recognition of the Gla region of FVII (amino acid residues 1 to 45 of human FVII). Although the immuno-affinity based purification step is highly selective and provides FVII protein of high purity, there are disadvantages to this step. For example, potential leaching of antibody into the drug product may affect the safety of the final drug, and the cost of producing the monoclonal antibody (mAb) immuno-affinity matrix is considerable as compared to more conventional, non-antibody based purification matrices.

While replacement of the immuno-affinity step with a different purification technique would be advantageous, this would require removal of non-FVII related contaminants as well as the ability to separate any unwanted isoforms of FVII that may be present in the culture supernatants.

### BRIEF DESCRIPTION OF THE DRAWINGS

Figure 1 shows a UV-trace of a FVII sample eluted from a hydroxyapatite column according to the invention as described in Example 1.
Figure 2 shows an SDS-PAGE analysis of the sample of Figure 1.
Figure 3 shows a UV-trace of a FVII sample eluted from a hydroxyapatite column according to the invention as described in Example 2.
Figure 4 shows an SDS-PAGE analysis of the sample of Figure 3.

### DESCRIPTION OF THE INVENTION

We have explored the possibilities of using non-antibody based methods for purification of FVII protein and possible separation of isoforms of FVII captured from the culture supernatants, and it has surprisingly been found that excellent results are obtained using hydroxyapatite. The present invention thus relates to a method suitable for purifying rFVII or rFVIIa, comprising subjecting the rFVII or rFVIIa to liquid chromatography on a hydroxyapatite (HAP) column, wherein the rFVII or rFVIIa is eluted as defined in claim 1.

### Definitions

In the description and claims below, the follow definitions apply:

The term "FVII" or "FVII polypeptide" refers to a FVII molecule provided in single chain form.

The term "FVIIa" or "FVIIa polypeptide" refers to a FVIIa molecule provided in its activated two-chain form, wherein the peptide bond between R152 and I153 of the single-chain form has been cleaved.

The terms "rFVII" and "rFVIIa" refer to FVII and FVIIa molecules produced by recombinant techniques, respectively. These may have the wild-type human sequence or may be variants of the human sequence.

The terms "hFVII" and "hFVIIa" refer to wild-type human FVII and FVIIa, respectively.

Unless it is indicated otherwise or apparent from the context, the terms "FVII", "FVII protein" and "Factor VII" as used herein are intended to include both the inactivated and activated forms of FVII, and to include the recombinant wild-type sequence of human FVII as well as variants thereof.

### Detailed description

The FVII proteins that may be purified by the method of the invention include any FVII or FVIIa protein, in particular human recombinant FVII or FVIIa and variants thereof. The amino acid sequence of wild-type human FVII is well known and is disclosed, for example, in WO 01/58935. Variants of interest that may be purified by the method of the invention include, for example, those described in WO 01/58935, WO 03/093465, WO 2004/029091, WO 2004/111242, WO 99/20767, WO 00/66753, WO 88/10295, WO 92/15656, WO 02/29025, WO 01/70763, WO 01/83725, WO 02/02764, WO 02/22776, WO 02/38162, WO 02/077218, WO 03/027147, WO 03/037932, WO 2004/000366, WO 2004/029090, and WO 2004/108763.

The FVII or FVIIa variants may include one or more substitutions, insertions or deletions compared to wild-type human FVII, for example resulting in a variant that differs in 1-15 amino acid residues from the amino acid sequence of wild-type human FVII, typically in 1-10 or in 2-10 amino acid residues, e.g. in 1-8 or in 2-8 amino acid residues, such as in 3-7 or in 4-6 amino acid residues from the amino acid sequence, where the differences in amino acid sequence from the wild-type are typically substitutions. Such substitutions may be performed e.g. with the aim of introducing one or more in vivo glycosylation sites or PEGylation sites into the protein and/or for improving or otherwise modifying the clotting activity of the wild-type protein; such variants are described in WO 01/58935, WO 03/093465, WO 2004/029091 and WO 2004/111242. FVII or FVIIa variants may alternatively have a reduced clotting activity in order to function as anti-coagulants.

The FVII/FVIIa protein or variant thereof may be produced by any suitable organism, e.g. in mammalian, yeast or bacterial cells, although eukaryotic cells are preferred, more preferably mammalian cells such as CHO cells, HEK cells or BHK cells.

Hydroxyapatite (HAP) is a porous inorganic chromatography material based on calcium phosphate that is useful for purification of proteins, including enzymes and monoclonal antibodies, as well as nucleic acids. Since HAP consists of calcium phosphate, it contains both positive and negative charges. Liquid chromatography using HAP is typically performed within a pH range of about 5.5-9, e.g. about 6-9.

The HAP column may be equilibrated with a low ionic strength buffer, e.g. about 5-10 mM, with a suitable buffer being e.g. a phosphate buffer such as sodium phosphate or alternatively a non-phosphate buffer such as imidazole, TRIS, histidine, MES (2-(N-morpholino)ethane sulfonic acid) or borate. The buffer may optionally include a salt such as NaCl.

The column itself may have any suitable size and volume, depending on e.g. the amount of protein and supernatant to be purified. Persons skilled in the art will be readily able to select a suitable column based on the actual purification needs.

It has been found that HAP is particularly well-suited for purification of rFVII as well as separation of unwanted FVII isoforms. The FVII protein binds tightly to HAP at low buffer concentrations, e.g. a phosphate concentration of from about 1 mM, such as from about 5 mm, e.g. from about 10 mM, and up to about 20 mM, at a pH of from about 5.5 to about 7.5, e.g. about 6.0-7.5, or in the absence of phosphate at a pH of from about 5.5 to about 9.0 or 9.5, typically about 6.0-S.6. The presence of a low concentration of CaCl₂, e.g. a concentration of up to about 1 mM, or a higher concentration, e.g. up to about 5, 10 or 20 mM, or even higher such as up to about 50 mM, does not prevent binding of the FVII protein to the HAP matrix.

Elution of non-FVII impurities and unwanted FVII isoforms, for example isoforms with an insufficient level of gamma-carboxylation in the Gla-region of FVII, can be achieved by use of mild elution conditions at a pH of from about 5.5 to about 7.5, e.g. washing with a phosphate concentration of from about 10 mM to about 50 mM or higher, such as up to about 100 or 150 mM, at a pH of about 6.0-7.0. A further alternative for elution of non-FVII impurities and unwanted FVII isoforms is use of a high concentration of NaCl, e.g. up to 1 M or even higher, such as up to about 1.5 M, in the presence of phosphate.

Elution of the FVII protein can be accomplished by increasing the phosphate concentration, the FVII protein being eluted from the column using an appropriate combination of pH and phosphate, for example a gradient starting at about 10-20 mM phosphate and increasing to a maximum phosphate concentration of e.g. about 400-500 mM at a pH of about 5.5 or higher, typically about 6.0 or higher, e.g. up to about 9.0 or 9.5, such as up to about 8.6. The phosphate concentration may be increased in either a linear or stepwise manner as is generally known in the art. Alternatively, elution may be performed in a stepwise manner or with a gradient using an increasing concentration of a non-phosphate displacer in a similar manner to elution with a phosphate buffer. In this case, the displacer could e.g. be calcium in the form of calcium chloride or calcium sulfate, with a pH of e.g. about pH 5.5-9.5, for example 6.0-9.0. Elution with phosphate will often be preferred.

Regeneration of the HAP column may be performed with a phosphate buffer, e.g. in a concentration of about 0.5 M.

Under certain elution conditions the FVII protein elutes in discrete peaks, possibly due to separation of different isoforms. In this case, different approaches are possible for separating the different fractions. One approach is to increase the phosphate or other displacer concentration as described above while maintaining a steady pH. After reaching the maximum phosphate/displacer concentration the pH can then be increased stepwise, typically by increasing the pH in a single step by a value of 1-3, e.g. about 1.5-2.5, such as about 2, over the pH used during the gradient elution. For example, the FVII protein may be eluted at pH of about 6-7 and a maximum concentration of phosphate or other displacer of 400-500 mM, after which the pH is increased from about 6-7 to about 8-9, e.g. from about 6 to about S or from about 7 to about 9, while maintaining the high phosphate/displacer level.

Alternatively, elution may be performed using a combined concentration and pH gradient in which both the phosphate/displacer concentration and the pH are increased simultaneously. This may take place starting with a low phosphate/displacer concentration of, e.g. about 10-20 mM, or after the phosphate/displacer concentration is initially raised to an intermediate level of e.g. about 100 or 150 mM. For example, after an initial increase of the phosphate concentration to an intermediate level of 100-150 mM, a phosphate concentration gradient starting at this level and increasing to e.g. about 400-500 mM can be used together with a pH gradient, typically starting with an initial pH of about 5.5-7.0 and increasing to a final pH of about 7.5-9.0, e.g. starting with a pH of about 6 and increasing to a pH of about 8. A variant of this approach is to use a stepwise elution in which the eluent concentration and/or the pH are altered so as to go directly from the intermediate level to final elution conditions. An example of such a stepwise elution is to go from a phosphate/displacer concentration of e.g. about 100 or 150 mM and a pH of e.g. about 6 in a single step to a phosphate/displacer concentration of e.g. about 400 or 500 mM and a pH of e.g. about 8 or 9. If desired, the combined phosphate/displacer and pH gradient can further be combined with a salt gradient, e.g. starting with a NaCl concentration of 0.5-1.5 M, such as about 1.0 M, and decreasing typically down to 0 M.

The FVII protein eluted by the method of the invention has been found to have a purity of greater than about 80%, and in some cases about 90% or more, as determined by LDS PAGE (lithium dodecyl sulfate-polyacrylamide gel electrophoresis).

The invention will be further described with reference to the following non-limiting examples.

### EXAMPLES

### Example 1

### Materials and methods

The recombinant human FVII protein applied onto the HAP column was produced in CHO-K1 cells. Culture supernatants were sterile-filtered, ultra-filtered and dia-filtered against 10 mM Tris pH 8.6. The sample was subsequently captured on a Q-Sepharose^{™} FF column previously equilibrated with 10 mM Tris, pH 8.6. After washing in 10 mM Tris, 100 mM NaCl, pH 8.6 the bound FVII protein was eluted in 10 mM Tris, 35 mM CaCl₂, 25 mM NaCl, pH 8.6. This sample was desalted to lower the conductivity in 20 mM Tris-HCl, 0.5 mM CaCl₂, pH 7.5 prior to application onto the hydroxyapatite column.

Ceramic hydroxyapatite type 1 was from Biorad (cat # 157-0400). The columns were packed at volumes of 3-10 ml with column diameters of 5 or 10 mm (Amersham Biosciences) in 0.2 M Na-Phosphate, pH 9-10 and subsequently equilibrated with 10 mM Tris, pH 8.6. The columns were run at room temperature and typically at up to 30 CV/h.

### Results

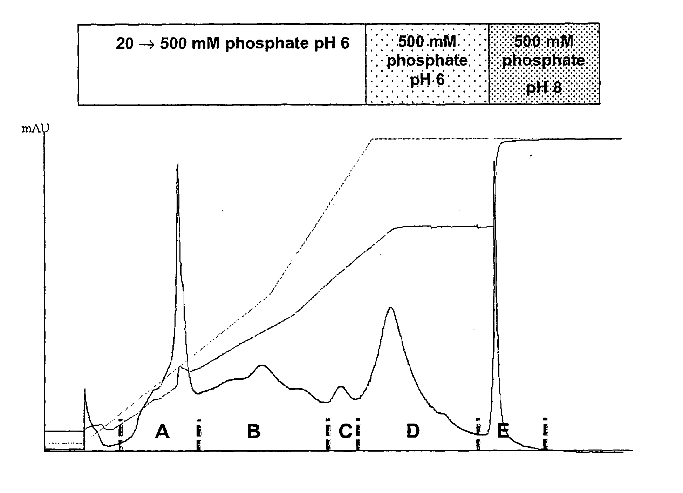

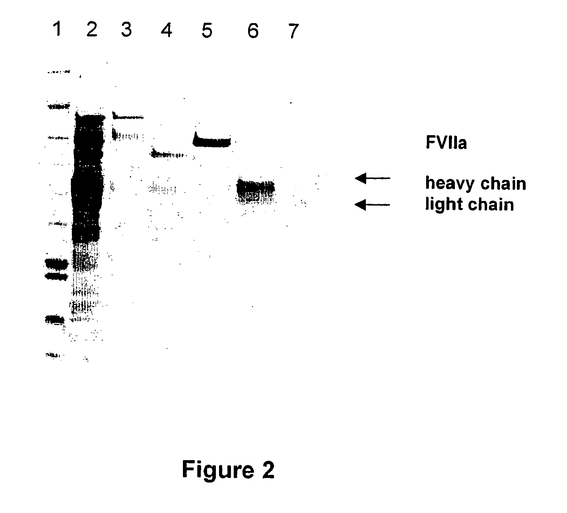

FVII eluted from an anion exchange capture as described above was bound in 20 mM Tris-HCl, 0.5 mM CaCl₂, pH 7.5 to the hydroxyapatite column. The bound protein was washed first in 20 mM Tris-HCl, pH 7.5, then using 20 mM Na-Phosphate, pH 6.0. Bound protein was then eluted with a gradient of 20-500 mM Na-Phosphate at pH 6.0 followed by a step of 500 mM Na-Phosphate pH 8.0. Figure 1 shows a UV-trace of sample eluted from the HAP column, where the letters A to E indicate pools that were analyzed by SDS-PAGE as shown in Figure 2, where Lane 1 is a MW standard, Lane 2 is the protein solution loaded unto the HAP-column, Lane 3 is pool A, Lane 4 is Pool B, Lane 5 is Pool C, Lane 6 is Pool D, and Lane 7 is Pool E. The highly selective elution of FVIIa at the end of a linear gradient of phosphate from 20 to 500 mM at pH 6.0 and at 500 mM phosphate pH 8.0 is shown in lanes 6 and 7, respectively.

The FVII protein eluted in two well-separated pools, one late in the gradient and one with the pH 8.0 step. The protein was >90% pure in both pools as estimated on SDS-PAGE (Figure 2).

### Example 2

### Materials and methods

The recombinant human FVII protein applied onto the HAP column was produced in CHO-K1 cells. Culture supernatants were sterile-filtered, ultra-filtered and dia-filtered against 25 mM imidazole, 25 mM NaCl, pH 7.0. 5 mM EDTA was subsequently added to the dia-filtered sample prior to capture on a Q-Sepharose^{™} FF column previously equilibrated with 25 mM imidazole, 25 mM NaCl, pH 7.0. After washing in 25 mM imidazole, 25 mM NaCl, 5 mM CaCl₂, pH 7.0, the bound FVII protein was eluted in 25 mM imidazole, 75 mM CaCl₂, 25 mM NaCl, pH 7.0. This sample was diluted in 25 mM imidazole, pH 6.5 to lower the conductivity prior to application onto the hydroxyapatite column.

Ceramic hydroxyapatite was as described above in Example 1. The columns were packed and run as described above, equilibrating with 25 mM imidazole, pH 6.5, with a column volume of 30 ml and a column diameter of 16 mm. The presence of FVII isomers is estimated by the ratio between two ELISAs, directed towards the Gla domain ("Sigma ELISA") and the PD domain (amino acid residues 153-406 of human FVII) ("PD ELISA"), respectively. The Sigma ELISA recognizes FVII protein with correctly folded and highly gamma-carboxylated Gla domains, and is thus a quantitative estimate of the population of FVII with functional Gla domains. The PD ELISA recognizes all FVII molecules in the sample irrespective of the condition of the Gla domain. The ratio of the two ELISAs is an indicator of the purity of the FVII population with a functional Gla domain.

### Results

FVII eluted from an anion exchange capture as described above was bound in 25 mM imidazole, approx. 6 mM NaCl, approx. 18 mM CaCl₂, pH 6.5 to the hydroxyapatite column. The bound protein was washed first in 25 mM imidazole, pH 6.5, then using 100 mM Na-Phosphate, pH 6.3, followed by 150 mM Na-Phosphate, 1 M NaCl to elute contaminants and unwanted FVII isoforms. FVII protein containing the desired highly gamma-carboxylated isoforms was then eluted using Na-Phosphate, 1 M NaCl, with a Na-Phosphate concentration gradient of from 150 mM to 500 mM together with a pH gradient of from 6.3 to 8.0.

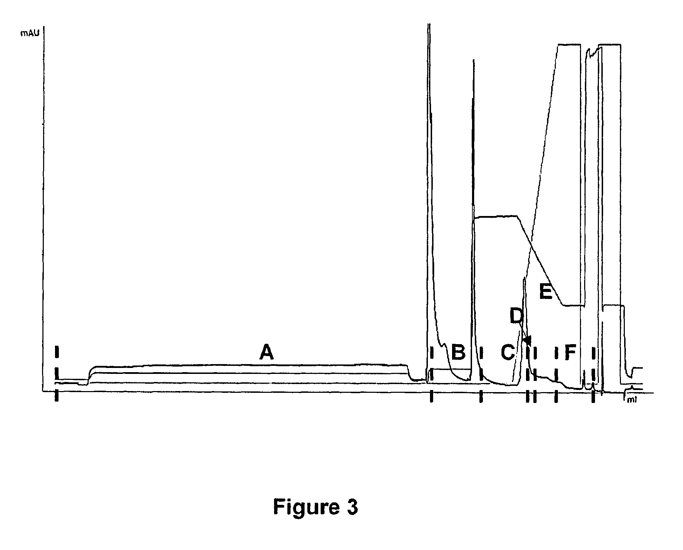

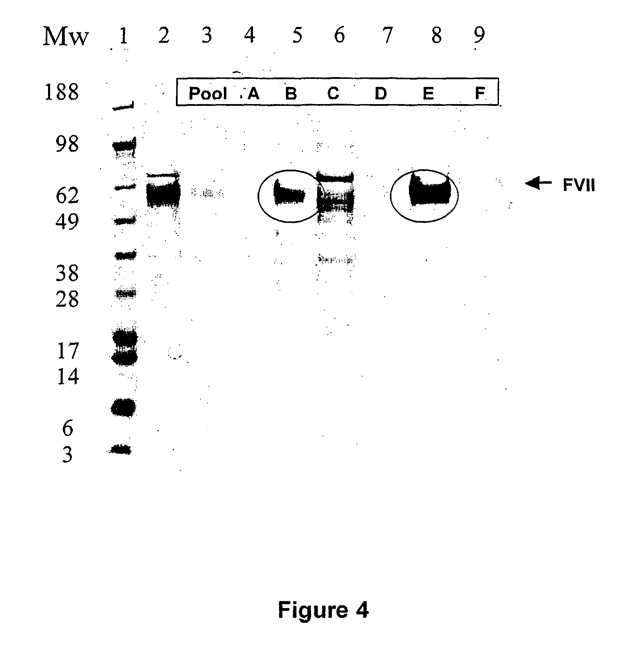

Figure 3 shows a UV-trace of sample eluted from the HAP column, where the letters A to F indicate pools that were analyzed by SDS-PAGE, where Lane 1 is a MW standard, Lane 2 is the starting material consisting of the capture eluate, Lane 3 is the diluted protein solution loaded unto the HAP column, Lane 4 is pool A (flow-through/wash), Lane 5 is Pool B (100 mM Na-Phosphate wash), Lane 6 is Pool C (150 mM Na-Phosphate, 1 M NaCl wash), Lane 7 is Pool D and Lane 8 is Pool E (where pools D and E comprise a combined linear phosphate/pH/salt gradient going from 150 mM to 500 mM Na-Phosphate, from pH 6.3 to pH 8.0, and from 1 M to 0 M NaCl, Pool D being the leading edge pre-elution and Pool E being the product pool), and lane 9 is Pool F (500 mM Na-Phosphate, pH 8.0, trailing edge post-elution). The highly selective elution of FVII using a linear gradient of Na-Phosphate concentration, pH and salt concentration may be seen in Lane 8 of Figure 4.

The FVII protein eluted under two different sets of conditions: one in moderate Na-Phosphate concentrations of up to 150 mM, with an NaCl concentration up to 1 M, at a pH between 6.0 to 6.5, and one where FVII is eluted in the combined phosphate/pH/salt gradient ending at 500 mM Na-Phosphate, and pH 8.0. The protein was >80% pure in the final product pool (Figure 4, lane 8)as estimated by SDS-PAGE. The Sigma:PD ELISA ratios of the eluted FVII pools were 0 (Figure 4, lane 5) and 1 (Figure 4, lane 8), respectively, suggesting a highly efficient removal of FVII protein with Gla domains that are unrecognizable by Sigma ELISA (i.e. with insufficiently gamma-carboxylated Gla domains) in the initial washes. This has also been confirmed by a quantifying Gla-anion exchange HPLC analysis.

## Claims

1. A method for purifying recombinant Factor VII (rFVII) or recombinant activated Factor VII (rFVIIa), comprising subjecting the rFVII or rFVIIa to liquid chromatography on a hydroxyapatite (HAP) column,
wherein the rFVII or rFVIIa is eluted from the HAP column using a linear or stepwise gradient of phosphate buffer or non- phosphate displacer with an increasing phosphate/displacer concentration,
and wherein either:
the method further comprises an elution step in which the pH is increased stepwise by a value of 1 -3 subsequent to the concentration gradient elution, or
the phosphate or displacer gradient is combined with a pH gradient with increasing pH.

2. The method of claim 1, wherein the rFVII or rFVIIa is human rFVII or rFVIIa.

3. The method of claim 1, wherein the rFVII or rFVIIa is a variant of human rFVII or rFVIIa, wherein the variant has an amino acid sequence that differs from the sequence of wild- type human FVII in 1-15 amino acid residues.

4. The method of any of the preceding claims, wherein the rFVII or rFVIIa is applied to the HAP column in a phosphate buffer having a pH of 5.5-7.5.

5. The method of any of claims 1 -3, wherein the rFVII or rFVIIa is applied to the HAP column in a non-phosphate buffer having a pH of 5.5-9.0.

6. The method of any of claims 1-5, comprising an initial elution step using a pH in the range of 5.5-7.5.

7. The method of claim 6, wherein the initial elution step is performed with phosphate in a concentration of up to about 150 mM.

8. The method ofany of the preceding claims, wherein elution is performed using a phosphate buffer with a pH in the range of 5.5-9.0.

9. The method of any of the preceding claims, wherein the pH is increased from 6-7 to 8-9.

10. The method of any of the preceding claims, wherein elution is performed using a phosphate buffer gradient and a pH gradient.

11. The method of claim any of the preceding claims, wherein the pH is increased from an initial pH of 5.5-7.0 to a final pH of 7.5-9.0.

## Patentansprüche

1. Verfahren zur Reinigung von rekombinantem Faktor VII (rFVII) oder rekombinantem aktiviertem Faktor VII (rFVIIa), umfassend das Unterziehen des rFVII oder rFVIIa einer Flüssigkeitschromatographie auf einer Hydroxyapatit- (HAP-) Säule, worin der rFVII oder rFVIIa aus der HAP-Säule unter Einsatz eines linearen oder schrittweisen Gradienten aus Phosphatpuffer oder einem nicht phosphathältigen Verdrängungsmittel mit steigender Phosphat-/Verdrängungsmittelkonzentration eluiert wird
und worin das Verfahren weiters entweder einen Elutionsschritt nach der Elution mit dem Konzentrationsgradienten umfasst, in dem der pH schrittweise um einen Wert von 1 bis 3 erhöht wird, oder der Phosphat- oder Verdrängungsmittelgradient mit einem pH-Gradienten mit steigendem pH kombiniert wird.

2. Verfahren nach Anspruch 1, worin der rFVII oder rFVIIa menschlicher rFVII oder rFVIIa ist.

3. Verfahren nach Anspruch 1, worin der rFVII oder rFVIIa eine Variante eines menschlichen rFVII oder rFVIIa ist, worin die Variante eine Aminosäuresequenz aufweist, die sich von der Sequenz von menschlichem Wildtyp-FVII in 1 bis 15 Aminosäureresten unterscheidet.

4. Verfahren nach einem der vorangegangenen Ansprüche, worin der rFVII oder rFVIIa auf die HAP-Säule in einem Phosphatpuffer mit einem pH von 5,5-7,5 aufgebracht wird.

5. Verfahren nach einem der Ansprüche 1 bis 3, worin der rFVII oder rFVIIa auf die HAP-Säule in einem nicht phosphathältigen Puffer mit einem pH von 5,5 bis 9,0 aufgebracht wird.

6. Verfahren nach einem der Ansprüche 1 bis 5, das einen anfänglichen Elutionsschritt unter Verwendung eines pH im Bereich von 5,5 bis 7,5 umfasst.

7. Verfahren nach Anspruch 6, worin der anfängliche Elutionsschritt mit Phosphat in einer Konzentration von bis zu etwa 150 mM durchgeführt wird.

8. Verfahren nach einem der vorangegangenen Ansprüche, worin die Elution unter Verwendung eines Phosphatpuffers mit einem pH im Bereich von 5,5 bis 9,0 durchgeführt wird.

9. Verfahren nach einem der vorangegangenen Ansprüche, worin der pH von 6 bis 7 auf 8 bis 9 erhöht wird.

10. Verfahren nach einem der vorangegangenen Ansprüche, worin die Elution unter Verwendung eines Phosphatpuffergradienten und eines pH-Gradienten durchgeführt wird.

11. Verfahren nach einem der vorangegangenen Ansprüche, worin der pH von einem Ausgangs-pH von 5,5 bis 7,0 auf einen End-pH von 7,5 bis 9,0 erhöht wird.

## Revendications

1. Procédé pour purifier du facteur VII recombinant (rFVII) ou du facteur VII recombinant activé (rFVIIa), comprenant l'exposition du rFVII ou du rFVIIa à une chromatographie liquide sur une colonne d'hydroxyapatite (HAP),
où le rFVII ou le rFVIIa est élué de la colonne de HAP au moyen d'un gradient linéaire ou par pas de tampon phosphate ou d'agent de déplacement non phosphate avec une concentration de phosphate/agent de déplacement croissante,
et où :
le procédé comprend en outre une étape d'élution dans laquelle le pH est augmenté par pas d'une valeur de 1-3 après l'élution en gradient de concentration, ou
le gradient de phosphate ou d'agent de déplacement est combiné avec un gradient de pH avec un pH croissant.

2. Procédé selon la revendication 1, où le rFVII ou le rFVIIa est du rFVII ou du rFVIIa humain.

3. Procédé selon la revendication 1, où le rFVII ou le rFVIIa est un variant de rFVII ou de rFVIIa humain, où le variant a une séquence d'aminoacides qui diffère de la séquence du FVII humain de type sauvage dans 1-15 résidus d'aminoacides.

4. Procédé selon l'une quelconque des revendications précédentes, où le rFVII ou le rFVIIa est appliqué à la colonne de HAP dans un tampon phosphate ayant un pH de 5,5-7,5.

5. Procédé selon l'une quelconque des revendications 1-3, où le rFVII ou le rFVIIa est appliqué à la colonne de HAP dans un tampon non phosphate ayant un pH de 5,5-9,0.

6. Procédé selon l'une quelconque des revendications 1-5, comprenant une étape d'élution initiale au moyen d'un pH dans la plage de 5,5-7,5.

7. Procédé selon la revendication 6, où l'étape d'élution initiale est accomplie avec du phosphate en une concentration pouvant atteindre environ 150 mM.

8. Procédé selon l'une quelconque des revendications précédentes, où l'élution est accomplie au moyen d'un tampon phosphate avec un pH dans la plage de 5,5-9,0.

9. Procédé selon l'une quelconque des revendications précédentes, où le pH est augmenté de 6-7 à 8-9.

10. Procédé selon l'une quelconque des revendications précédentes, où l'élution est accomplie au moyen d'un gradient de tampon phosphate et d'un gradient de pH.

11. Procédé selon l'une quelconque des revendications précédentes, où le pH est augmenté d'un pH initial de 5,5-7,0 à un pH final de 7,5-9,0.
